# EUROPEAN PATENT SPECIFICATION

(11) **EP 3 443 990 B1**
(45) Date of publication and mention of the grant of the patent: **30.09.2020**
(21) Application number: 17788509.2
(22) Date of filing: 13.02.2017
(51) Int. Cl.: A61L 27/36

(54) **COMPOSITE EXTRACELLULAR MATRIX BIOMATERIAL**
ZUSAMMENGESETZTES BIOMATERIAL FÜR EXTRAZELLULÄRE MATRIX
BIOMATÉRIAU COMPOSITE DE TYPE MATRICE EXTRACELLULAIRE

(30) Priority: 25.04.2016 CN 201610261472
(43) Date of publication of application: 20.02.2019
(73) Proprietor: Shanghai Zhuoruan Medical Technologies Co., Ltd, Shanghai 200233 (CN); Zhuoruan Medical Technologies (Suzhou) Co., Ltd, Taicang, Jiangsu 215434 (CN)
(72) Inventor: ZHANG, Jian, Jiangsu 215434 (CN)
(74) Representative: EP&C
(86) International application number: PCT/CN2017/073394
(87) International publication number: WO 2017/185853

(56) References cited:
- EP-A1- 1 177 800
- WO-A1-2009/070686
- CN-A- 101 361 989
- CN-A- 101 366 979
- CN-A- 105 920 669
- US-A1- 2011 020 420
- US-A1- 2014 309 739

## Description

### TECHNICAL FIELD

The present invention relates to the field of materials for tissue repair, in particular, a biological material with composite extracellular matrix component

### BACKGROUND OF THE INVENTION

Acellular tissue matrix (ACTM) is an important progress made in the study on the material for soft tissue repair in recent two decades. Cells, antigens, lipids, soluble proteins, and the like in raw tissues are removed completely by physical or chemical methods, and the resulted insoluble extracellular matrix (ECM) with highly preserved appearance, histological characteristics, and ultrastructure is used as biological scaffold, namely ACTM. ECM is highly conserved in the process of biological evolution, and ECM of the same tissues slightly differs among different species. Therefore, ACTM deprived of cell components and antigens that may cause immunological rejections by decellularization process can be used safely as allografts and xenografts. When used to repair tissue defects, synthetic material induces chronic inflammatory stimulations that recruit fibroblasts to form scars; differing from the aforesaid mechanism, ACTM can induce endogenous tissue regeneration, i.e., biological signals and degradation products from the implanted ACTM can induce macrophages and stem cells locates around the region under repair to infiltrate scaffold, to grow and proliferate rapidly, and to secrete their own extracellular matrix to substitute implants. Host tissue grows in ACTM while the later degrades gradually, these two processes are simultaneous basically, and in the end ACTM will be replaced completely by host tissue. Therefore ACTM as the material for tissue repair manifests more advantages: ① Its structure and components are close to those of natural tissues: its main components are collagen fibers and other structural proteins, with trace glycoprotein, fibronectin, glycosaminoglycan and proteoglycan, growth factors, enzymes, etc., ② ACTM shows reasonable and controllable degradability, which accords better with the pattern of regeneration; ③ ACTM possesses reasonable porosity, which benefits the interchange of substances and air among tissues; ④ACTM possesses reasonable mechanical strength, so that it can support the ingrowth of tissues; ⑤ACTM possesses tolerance to infection, and therefore it can be used to repair contaminated or potentially contaminated tissue defects, which may be attributed to timely ingrowth of phagocytes, early and local revascularization, so that bacterial biofilm is hard to form.

At present, ACTM is clinically used as substitutes for meninx, pleura, abdominal wall, fascia, etc., as well as used in stoma reinforcement, pelvic fundus reconstruction, urinary bladder suspend, injured solid viscera (such as spleen, liver) packing hemostasis, and treatments of various complex hernias and abdominal wall defects, such as contaminated abdominal wall defects, infection after implantation of synthetic materials, reoperation of intestinal fistula, etc. Statistically, ACTM accounted for 5∼10% of all materials used in the repair of soft tissue defects in US. Biological mesh can be divided into following two main types depending on the source of raw materials: ① Materials from inert tissue (IT), represented by human/porcine dermis, bovine/porcine/equine pericardium, and bovine/porcine peritoneum. These materials are derived from biologically inert tissues in the body, consisted of almost only structural proteins (comprises collagen fibers and elastic fibers), and absence of bioactive components such as fibronectin, growth factors, and proteoglycan. ②Biological materials from ECM, possess intact 3D-ultrastructure of extracellular matrix from live tissues and bioactive components such as fibronectin, growth factors, and glycosaminoglycan, represented by small intestinal submucosa (SIS), human amniotic membrane, and urinary bladder matrix (UBM). The technology for preparing biological materials of ECM sourced (including decellularization and shaping) is more complex than that of IT tissue source, however, the former is superior to the later in terms of the repair efficacy of tissue defects: ①The implantation of IT sourced materials can only induce the regeneration of vascularized connective tissues to fill in and anatomically repair the defects, while ECM biological materials can integrate effectively with host tissue, actively induce autologous stem cells to migrate to the injured site, and promote their proliferation and differentiation to realize tissue-specific and functional repair to some degrees; for example, ECM biological materials can be used to realize the regeneration of muscle and fascia, partial recovery of innervation to improve the function of disabled extremities, and reconstruction of fingertips, etc. ② Materials of IT source are dense in structure and contain a lot of elastic fibers that degrade slowly and cannot autologous renew after the age of 25, leading to long-term instability and loss of elasticity in implanted regions. ③In contrast with biological materials of IT source, those of ECM source possess better tolerance to infection and faster tissue regeneration.

SIS is obtained from mammalian small intestine that is delaminated mechanically to remove serosa, mucosa and muscularis layers and then decellularized. SIS is mainly consisted of type-I and type-III collagen, and trace type-IV and type-V collagen, glycosaminoglycan, growth factor, fibronectin, etc. Implanted SIS can degrade completely, and therefore it is a favorable scaffold in tissue engineering. SIS possesses good mechanical strength, broad source of raw materials, and easier or automated pre-treatment. However, its bioactivity is relatively low, and it carries higher original bioburdens because its raw material may contact various antigens in food in vivo, therefore these bioburdens (likely endotoxin, Gal epitope residue) reserve immunogenicity even after harsh decellularization and sterilization, which causes host responses.

Small intestinal submucosa has been used in the manufacture of products described in several inventions. US Patent 2011/020420 describes a tissue graft comprising at least two layers, at least one layer comprising an ECM having a density that differs from at least one of the other layers, each of the layers having a first planar surface and a second planar surface and each layer at least partially overlapping an adjacent layer. The ECM may be SIS, UBS (urinary bladder submucosa) or UBM. A less dense layer that is bioabsorbed more quickly, thereby allowing rapid cellular infiltration and vascularization of the tissue graft composition and release of bioactive factors. A layer of high density may provide a strong support composition to withstand forces and tear or rupture at the treatment site.

European Patent 1177800 describes a bioprosthetic device comprising a top layer of SIS and a bottom layer of SIS, and a synthetic mesh device positioned to lie between top and bottom layers of SIS. The layers may be sutured or tacket together. Instead of SIS other sources of ECM may be used, e.g. bladder.

PCT Patent WO2009/070686 described a device for plugging a passageway in the body comprising a sheet-form material, preferably comprising a plurality of layers of ECM materials. Different ECM materials may be combined. A suitable ECM material comprising SIS, stomach submucosa, UBS.

However, the products these inventions dose not reduce the immunogenicity of SIS.

China Patent No. CN2608014 disclosures an artificial dura mater, which is prepared by bonding small intestine submucosa to human amniotic membrane in order that it can have the mechanical tensile strength of human dura mater and anti-adhesion ability of arachnoid membrane both. However, the aforesaid method of preparation dose not completely isolate the immunogenicity of SIS.

China Patent No. CN101366979 disclosures a tissue patch and related methods of preparation; in the said tissue patch, decellularized small intestine submucosa is used as internal layer that is encapsulated with decellularized amniotic membrane. Decellularized amniotic membranes isolated the immunogenicity of decellularized small intestine submucosa, and decellularized small intestine submucosa reinforced the mechanical strength of decellularized amniotic membranes. The said tissue patch shows higher bioactivity and histocompatibility, meantime no significant immunological rejection or cytotoxicity. However, the amniotic membrane is a material of human origin, so that its source is difficult to control; in addition, it carries the risk of transmitting unknown viruses or diseases.

UBM is obtained from mammalian urinary bladder that is treated mechanically to remove serosa, muscularis external, submucosa, and muscularis mucosa layers and then decellularized. In contrast with SIS, UBM shows following advantages: ①Very low immunogenicity and high histocompatibility: UBM do not contact the bioburden such as bacteria *in vivo* with simple hierarchy structure, so that endotoxin contamination can be avoided in the process of harvest. ②High bioactivity: UBM contains intact structure and components of basement membrane, constructs to support the growth of epithelial cells of blood vessels and skin, and promotes tissue-specific cells to form continuous layers to inhibit the formation of scar, which facilitates tissue repair and wound healing, realizes the tissue-specific regeneration. For patients who used Matristem (UBM product manufactured by Acell Inc.), the healing time for open wounds was shortened from 25.5 weeks to 9.8 weeks. Degradation products of UBM contain over 5000 kinds of active components, of which 41 kinds of proteins or polypeptides have been proven to be associated with wound healing, their functions including nourishing nerves, promoting revascularization, and inhibiting tumor growth, etc. (Table 1). In the process of production, however, the delamination of UBM costs a lot of labor; in addition, it is difficult to fabricate UBM into products of proper thickness for its unsatisfied mechanical strength and smooth surface.

**Table 1 Active components of degradation products of UBM**

| Protein Name *(b)* | Functional group *(c)* | subcellular location *(d)* | Molecular weight (kDa) *(e)* | Number of unique polypeptides *(h)* |
|---|---|---|---|---|
| Collagen α-3(VI) chain | Adhesion | Extracellular matrix | 343.6 | 8 |
| Collagen α-1(XIV) chain | Adhesion | Extracellular matrix | 193.5 | 4 |
| Vinculin | Adhesion | Cytoplasm | 123.9 | 11 |
| Collagen α-2(VI) chain | Adhesion | Extracellular matrix | 108.6 | 2 |
| Vimentin | Adhesion | Cytoplasm | 53.7 | 9 |
| Laminin subunits γ-1 | Adhesion | Extracellular matrix | 177.6 | 5 |
| TGF-β-induced protein ig-h3 | Adhesion | Secreted | 74.4 | 2 |
| Collagen α-1(III) chain | Adhesion | Extracellular matrix | 93.6 | 3 |
| Periosteal protein | Adhesion | Extracellular matrix | 93.1 | 3 |
| Fibula protein-5 | Adhesion | Extracellular matrix | 50.1 | 2 |
| Palladin | Adhesion | Cytoskeleton | 150.5 | 2 |
| Dermatopontin | Adhesion | Extracellular matrix | 24 | 2 |
| Desmuslin | Adhesion | Cytoplasm | 172.7 | 3 |
| Collagen α-1(VI) chain | Adhesion | Extracellular matrix | 108.5 | 3 |
| Collagen binding protein 2 | Adhesion | Extracellular matrix | 46.4 | 2 |
| Nestin -2 | Adhesion | Extracellular matrix | 154.2 | 2 |
| Annexin A5 | Anti -apoptosis | Cytoplasm | 35.9 | 6 |
| Gelsolin | Anti-apoptosis | Cytoplasm | 80.7 | 5 |
| Dermcidin | Resistance to bacteria | Secretion | 11.3 | 4 |
| Caspase -14 | Differentiation | Cytoplasm | 27.7 | 4 |
| Neuroblast differentiation-associated protein AHNAK | Differentiation | Nuclei | 629.1 | 7 |
| Mimecan | Growth | Secretion | 34.2 | 4 |
| Galectin-1 | Growth | Extracellular matrix | 14.7 | 2 |
| Dihydropyrimidinase-related protein 3 | Growth | Cytoplasm | 61.9 | 2 |
| Galectin- 7 | Growth | Extracellular matrix | 14.9 | 2 |
| Obscurin | Growth | Cell membrane | 867.9 | 7 |
| Serum albumin | Regulatory | Secretion | 69.7 | 4 |
| Annexin A6 | Regulatory | Cytoplasm | 75.9 | 8 |
| Tubulin α-1A chain | Regulatory | Cell skeleton | 50.1 | 8 |
| Myosin modulatory light polypeptide 9 | Regulatory | Cytoplasm | 19.8 | 5 |
| Calponin-1 | Regulatory | Cytoplasm | 33.2 | 5 |
| Integrin β-1 | Regulatory | membrane | 88.1 | 5 |
| Myosin light polypeptide | Regulatory | unspecific | 16.9 | 6 |
| Myosin light polypeptide 6 | Regulatory | Cell skeleton | 16.9 | 2 |
| Peroxiredoxin-1 | Regulatory | Cytoplasm | 22.1 | 3 |
| Interleukin enhancer-binding factor 2 | Regulation | Nucleus | 43 | 2 |
| Histone H3.1t | Remodeling | Nucleus | 15.5 | 2 |
| Fibronectin | Wound healing | Secretion | 262.6 | 9 |
| Decorin | Wound healing | Extracellular matrix | 39.9 | 2 |
| Homerin | Wound healing | Cytoplasm | 282.4 | 3 |
| Lumican | Wound healing | Secretion | 38.7 | 2 |

### SUMMARY OF THE INVENTION

The scope of this invention is defined by the claims. Independent claim 1 relates to a biological material with composite extracellular matrix components, comprising an interlayer containing a decellularized small intestinal submucosa (SIS), a superior surface layer containing a decellularized urinary bladder matrix (UBM), an inferior surface layer containing a UBM, wherein the superior surface layer and the inferior surface layer encapsulated completely the interlayer to form a sandwich structure. The present invention aims to solve a technical problem as to how to provide a biological material with composite extracellular matrix component that integrates merits of UBM and SIS both: UBM isolates the immunogenicity of SIS and the direct contact of SIS with host tissues; after the implantation of the said biological material, the basic inflammatory response in the host-implant marginal zone is the same as that of pure UBM, with high biocompatibility; in addition, SIS can make up the disadvantage of UBM-low mechanical strength; furthermore, the preparation of SIS is easier, and its thickness can be changed by composition. Therefore, the said biological material can be used in the filling, reinforcement, repair or reconstruction of fascia, meninx, pleura, pelvic fundus, derma, solid viscera and various soft tissue defects, possessing good clinical practicability.

The present invention relates to a biological material with composite extracellular matrix components, wherein decellularized small intestinal submucosa (SIS) is used as interlayer of the biological material, decellularized urinary bladder matrix (UBM) is used as a superior surface layer and the inferior surface layer of the biological material, and the superior surface layer and the inferior surface layer encapsulated completely the interlayer to form a sandwich structure.

The SIS is obtained from mammalian small intestine that is delaminated mechanically to remove serosa, mucosa and muscularis layers and then decellularized, and is a membrane-like material.

The UBM is obtained from mammalian urinary bladder that is delaminated mechanically to remove serosa, muscularis external, submucosa and muscularis mucosa layers and then decellularized, and is a membrane-like material.

A number of layers in the said interlayer is 1∼20.

A number of layers in the said superior or inferior surface layers is 1∼10 respectively.

A interlayer is bonded between the superior surface layer and the inferior surface layer by one or several of such methods as medical adhesive, suturing and tying, and vacuum pressing; layers in interlayer, superior and inferior surface layers (among multilayered UBM and multilayered SIS) are bonded among them with the aforesaid method too.

The superior surface layer and the inferior surface layer possess high bioactivity, and they can isolate effectively the immunogenicity of interlayer and do not change the type of inflammatory responses of pure UBM in the marginal zone of host tissues. The interlayer can improve remarkably the mechanical strength and thickness of the said biological material.

The medical adhesive is one or several of chitosan, collagen, fibrin glue, hyaluronic acid, chondroitin sulfate, hydrogel, bone glue, gelatin, or pectin. Preferably, absorbable medical adhesives and sutures.

A technical parameter of the said vacuum pressing is Vacuum pressure: -50 ∼ -760 mmHg, acting duration: 0.5∼72h.

The biological material comprises also perforations that penetrate the biological material.

A diameter of the perforations is 1∼5 mm, and a spacing between the perforations is 0.5∼5 cm.

### EFFECTS OF THE INVENTION

(1) High bioactivity, slight tissue adhesion and no excessive scars; intact basement membrane components of the surface layer can release a lot of active factors to support and regulate such live activities as growth and differentiation of cells; for example, released basic fibroblast growth factor, epithelial growth factor, hepatocyte growth factor, and keratin growth factor to promote cell adhesion and migration, induce cell differentiation, and reduce cell apoptosis; it regulates the infiltration of epithelial cells to precede that of fibroblasts, inhibits the secretion of excessive fibrinogen, thus epithelial tissue will be formed before fibroblasts predominate, and the smooth surface of basement membrane of the said biological material helps to reduce adhesion and inhibit the formation of scar tissue.
(2) High histocompatibility, low immunogenicity, and same basic type of inflammatory response as pure UBM in the host-implant marginal zone: SIS is encapsulated by UBM with very low immunogenicity, so that SIS cannot directly contact host tissue in the early period of implantation to retard the release of components that may cause immune responses, and the type and severity of immune responses of the said biological material would be the same as those of pure UBM. The degradation of UBM and tissues ingrowth are simultaneous, and the gradual exposure of SIS in the late period of implantation would not change the results of tissue repair. Holes penetrating all layers of said material speed up the ingrowth of cells from host tissues, ease the flow of tissue fluid, and therefore result in decreased incidence of local complications such as seroma.
(3) Good mechanical strength, flexible thickness, and suitability for the repair of different wounds: The interlayer may enhance the mechanical strength of the said biological material, and number of layers of interlayer can be adjusted according to mechanical strength of defects. The combined application of medical adhesives and vacuum pressing enhances the peeling strength of the said biological material, so that the multilayered biological material is difficult to delaminate and can keep intact after hydration and composition.
(4) Decreased difficulty in the pretreatment of raw materials and lower price of the product: While combining merits of both UBM and SIS, the present invention can greatly reduce the usage of UBM, which is a limiting factor to yield and cycle of production, thus the cost of raw materials can be lowered, the cycle of production shortened, and the input of human labor reduced.

### BRIEF DESCRIPTION OF THE DRAWINGS

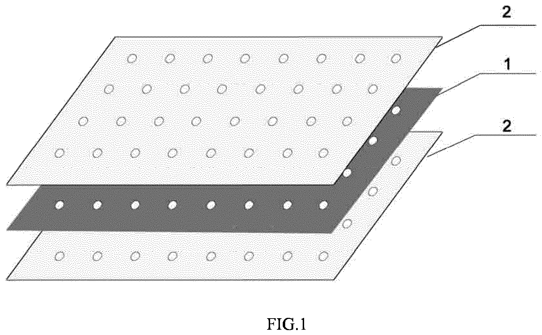

FIG. 1 shows the structure of the present invention, wherein digit 1 indicates decellularized small intestinal submucosa (SIS), and digit 2 indicates decellularized urinary bladder matrix (UBM)

### DETAILED DESCRIPTION OF THE PREFERRED EMBODIMENT

Following specific embodiments are used to further expound the present invention. Those embodiments are only for explaining the present invention, and shall not limit the scope of the present invention.

### Example 1

Use Abraham method to prepare porcine decellularized urinary bladder matrix (UBM) and decellularized small intestinal submucosa (SIS): Spread a monolayered UBM out smoothly (the smooth surface being downward), composite SIS into an independent layer, each SIS being overlapped by 50%. Then place 4 aforesaid independent layers on the aforesaid UBM, each layers being interlaced by 90°. Place a monolayered UBM on the surface of the aforesaid layers (the smooth surface being upward). Dissipate bubbles, bind all interlayers with medical chitosan as adhesive, then press the above layers under -250mm Hg for 24 h to make it become a whole material. The aforesaid material is perforated through all layers, the hole spacing being 5 mm, and the diameter of hole being 1 mm.

### Example 2

Use Abraham method to prepare porcine decellularized urinary bladder matrix (UBM) and decellularized small intestinal submucosa (SIS): Spread 2 layers of UBM out smoothly (the smooth surface being downward), composite SIS into an independent layer, each SIS being overlapped by 50%, then place 6 aforesaid independent layers on the surface of UBM, each layers being interlaced by 90° . Spread 2 layers of UBM on the surface of the aforesaid layers (the smooth surface being upward). Dissipate bubbles, bind all interlayers with medical collagen as adhesive, then press the above layers under -300mm Hg for 36 h to make it become a whole material. The aforesaid material is perforated through all layers, the hole spacing being 8 mm, and the diameter of hole being 2 mm.

### Example 3

According to GB/T528-2009, 3 samples were taken, each being 4 cm×1 cm in size and dumbbell-like in shape; aforesaid 3 samples were hydrated and then their two ends were fixed to a mechanical tester and pulled at the speed of 10 mm/min, and the tensile strength of those samples was 34 + 3 N/cm.

Three samples were taken and cut into 2 cm×5 cm in size; two ends of those samples were fixed to upper and lower clips of a tensile machine respectively, and those samples were peeled continuously at the speed of 10 mm/min till the overlapped part of them laminated; the force at stratification was recorded. The peeling strength of SIS-SIS and UBM-SIS was 6±2 N/cm, and the force for maintaining peeling was 1.5 + 0.5 N/cm.

The cytotoxicity of the said material was evaluated by the method claimed in GB/T 16886.5. NIH3T3 cells and L929 cells were used, and cell culture medium was used as extracting agent, extracts of gradient concentrations were used as cell culture media, and MTT method was used to determine the cell viability. The cytotoxicity of the said biological material was graded to be 0∼1.

Cell migration: The said material was powered under low temperature and then degraded by protease, the concentration of enzymatic products being 50 µg/mL. Cells were starved for 24h, and Boyden chamber method was used to determine 6h-migration of cells, medium with and without 10% fetal bovine serum being used as positive and negative control respectively. Migrated cells for the said material was 2056±72, and that for positive control was 2105±35, that for negative control was 1328±65. No significant difference was detected between the said material and positive control regarding cell migration (*P >* 0.05).

Endotoxin content of the said biological material was determined by the method claimed in GB/T14233.2. The water for endotoxin test was used as extracting agent, and the extraction was performed at 37□ for 24h. Endotoxin content was determined by kinetic turbidimetric limulus tests, and the endotoxin concentration of diluted extract was re-determined to rule out the interference. The endotoxin content of the said material was≤5EU/device.

The hemocompatibility of the said material was determined by the method claimed in GB/T14233.2. Contact group: The back of rats was dehaired, applied with 50µg/mL enzymatic products for once a day, consecutive 20 days. Oral administration group: 1 ml of extract was administered orally every other day within 7 days, 4 administrations in total; intramuscular injection and intravenous injection group: 0.15 mL of extract was injected every other day within 7 days, 4 injections in total. Rats in aforesaid 4 groups were killed 30 days and 90 days respectively after the administration, and the venous blood was collected for detection. The hemolytic ratio was calculated by the following formula: Hemolytic rate (%) = (absorbance of sample minus absorbance of negative control) divided by (absorbance of positive control minus absorbance of negative control) × 100 %. The hemolytic rate of the said material was≤5%.

The intradermal stimulation of the said material was evaluated by the method claimed in GB/T 16886.10. Rabbits were subject to the intradermal injection of 0.2 mL of extract and 0.2 mL of control (PBS) respectively, and the skin reaction of the injected region was observed 15 min, 1h, 2d, and 3d after the injection; the stimulation grades was scored as erythema and edema. The said material showed no intradermal stimulation.

The sensitization of the said material was evaluated by the maximal dose method claimed in GB/T 16886.10. The solution of pure starch was used as negative control. Extracts of the said material and control were oral administered consecutively for one week. Rats were observed for one week after oral administration. Weight, clinical toxic symptoms and grade of toxicity were daily recorded. All rats were killed after the test, and the pathological examination demonstrated that the said material showed no delayed super-sensitivity.

The animal model with defects of rectus abdominis sheath and rectus abdominis was established in dogs, the defect area being 10×5 cm²; the said material was cut into suitable size for defect repair, pure SIS and pure UBM being used as controls. The incidence of seroma in the repair region was 33% for pure SIS, and no seroma occurred for pure UBM and the said material. Repair region was harvested 2 weeks, 1 month, 2 months, and 4 months respectively after the aforesaid repair and subject to staining of CD68, CCR7, and CD163 to observe the classification and density of infiltrated cells and the ratio of M1 macrophages to M2 macrophages. It was confirmed that the basic type of inflammatory interaction of said material in the host-implant marginal zone was the same as that of pure UBM, and repair efficiency was close to that achieved by pure UBM.

## Claims

1. A biological material with composite extracellular matrix components, comprising:
an interlayer containing a decellularized small intestinal submucosa (SIS);
a superior surface layer containing a decellularized urinary bladder matrix (UBM);
an inferior surface layer containing a UBM;
wherein the superior surface layer and the inferior surface layer encapsulated completely the interlayer to form a sandwich structure.

2. A biological material with composite extracellular matrix components of claim 1, wherein the SIS is obtained from mammalian small intestine that is delaminated mechanically to remove serosa, and muscularis layers and then decellularized.

3. A biological material with composite extracellular matrix components of claim 1, wherein the UBM is obtained from mammalian urinary bladder that is treated mechanically to remove serosa, muscularis external, submucosa, and muscularis mucosa layers and then decellularized.

4. A biological material with composite extracellular matrix components of claim 1, wherein the number of layers in the interlayer is from 1 to 20.

5. A biological material with composite extracellular matrix components of claim 1, wherein each of the superior surface layer and the inferior surface layer comprises from 1 to 10 layers.

6. A biological material with composite extracellular matrix components of claim 4 or 5, wherein the said interlayer is bonded to the superior surface layer and the inferior surface layer by one or several methods including but not limited to medical adhesive, suturing and tying, and vacuum pressing;
wherein the layers in interlayer, superior surface layer and inferior surface layer are bonded among them with the aforesaid method too.

7. A biological material with composite extracellular matrix components of claim 6, wherein said medical adhesive comprises one or several of chitosan, collagen, fibrin glue, hyaluronic acid, chondroitin sulfate, hydrogel, bone glue, gelatin, and pectin.

8. A biological material with composite extracellular matrix components of claim 1, wherein the biological material comprises perforations that penetrate the biological material.

9. A biological material with composite extracellular matrix components of claim 8, wherein the perforations have a diameter of 1mm to 5 mm,
wherein the spacing between the perforations is 0.5cm to 5 cm.

## Patentansprüche

1. Ein biologisches Material mit zusammengesetzten extrazellulären Matrixkomponenten, umfassend:
eine Zwischenschicht, die eine dezellularisierte Dünndarmsubmukosa (SIS) enthält;
eine überlegene Oberflächenschicht, die eine dezellularisierte Harnblasenmatrix (UBM) enthält;
eine untere Oberflächenschicht, die ein UBM enthält;
wobei die obere Oberflächenschicht und die untere Oberflächenschicht die Zwischenschicht vollständig einkapselten, um eine Sandwichstruktur zu bilden.

2. Biologisches Material mit zusammengesetzten extrazellulären Matrixkomponenten nach Anspruch 1, wobei das SIS aus Dünndarm von Säugetieren erhalten wird, das mechanisch delaminiert wird, um Serosa- und Muscularis-Schichten zu entfernen, und dann dezellularisiert wird.

3. Biologisches Material mit zusammengesetzten extrazellulären Matrixkomponenten nach Anspruch 1, wobei das UBM aus der Harnblase von Säugetieren erhalten wird, das mechanisch behandelt wird, um Serosa-, Muscularis-Extern-, Submucosa- und Muscularis-Mucosa-Schichten zu entfernen und dann dezellularisiert wird.

4. Biologisches Material mit zusammengesetzten extrazellulären Matrixkomponenten nach Anspruch 1, wobei die Anzahl der Schichten in der Zwischenschicht 1 bis 20 beträgt.

5. Biologisches Material mit zusammengesetzten extrazellulären Matrixkomponenten nach Anspruch 1, wobei jede der oberen Oberflächenschicht und der unteren Oberflächenschicht 1 bis 10 Schichten umfasst.

6. Biologisches Material mit zusammengesetzten extrazellulären Matrixkomponenten nach Anspruch 4 oder 5, wobei die Zwischenschicht durch ein oder mehrere Verfahren mit der oberen Oberflächenschicht und der unteren Oberflächenschicht verbunden ist, einschließlich, aber nicht beschränkt auf medizinisches Kleben, Nähen und Binden und Vakuumpressen; wobei die Schichten in der Zwischenschicht, der oberen Oberflächenschicht und der unteren Oberflächenschicht auch mit dem vorgenannten Verfahren untereinander verbunden sind.

7. Biologisches Material mit zusammengesetzten extrazellulären Matrixkomponenten nach Anspruch 6, wobei der medizinische Klebstoff eines oder mehrere von Chitosan, Kollagen, Fibrinkleber, Hyaluronsäure, Chondroitinsulfat, Hydrogel, Knochenkleber, Gelatine und Pektin umfasst.

8. Biologisches Material mit zusammengesetzten extrazellulären Matrixkomponenten nach Anspruch 1, wobei das biologische Material Perforationen umfasst, die das biologische Material durchdringen.

9. Biologisches Material mit zusammengesetzten extrazellulären Matrixkomponenten nach Anspruch 8, wobei die Perforationen einen Durchmesser von 1 mm bis 5 mm haben, wobei der Abstand zwischen den Perforationen 0,5 cm bis 5 cm beträgt.

## Revendications

1. Matériau biologique à composants composites à matrice extracellulaire, comprenant:
une couche intermédiaire contenant une petite sous-muqueuse intestinale décellularisée (SIS);
une couche superficielle supérieure contenant une matrice de vessie urinaire décellularisée (UBM);
une couche de surface inférieure contenant un UBM;
dans lequel la couche superficielle supérieure et la couche superficielle inférieure encapsulées complètement l'intercalaire pour former une structure sandwich.

2. Matériau biologique avec des composants de matrice extracellulaire composite selon la revendication 1, dans lequel le SIS est obtenu à partir de l'intestin grêle de mammifère qui est délaminé mécaniquement pour éliminer séreuses et musculaires, puis décellularisées.

3. Matériau biologique à composants de matrice extracellulaire composite selon la revendication 1, dans lequel l'UBM est obtenu à partir de la vessie des mammifères qui est traitée mécaniquement pour éliminer séreuse, musculaire externe, sous-muqueuse et musculaire, puis décellularisée.

4. Matériau biologique avec des composants de matrice extracellulaire composite selon la revendication 1, dans lequel le nombre de couches dans la couche intermédiaire est de 1 à 20.

5. Matériau biologique avec des composants de matrice extracellulaire composite selon la revendication 1, dans lequel chacune de la couche superficielle supérieure et de la couche superficielle inférieure comprend de 1 à 10 couches.

6. Matériau biologique à composants de matrice extracellulaire composite selon la revendication 4 ou 5, dans lequel ladite couche intermédiaire est liée à la couche de surface supérieure et à la couche de surface inférieure par une ou plusieurs méthodes, y compris, mais sans s'y limiter, la colle médicale, la suture et le liage, et pressage sous vide;
dans lequel les couches en couche intermédiaire, couche superficielle supérieure et couche superficielle inférieure sont liées parmi eux avec la méthode susmentionnée aussi.

7. Matériau biologique avec des composants de matrice extracellulaire composite selon la revendication 6, dans lequel ledit adhésif médical comprend un ou plusieurs de chitosane, collagène, colle de fibrine, hyaluronique acide, sulfate de chondroïtine, hydrogel, colle osseuse, gélatine et pectine.

8. Matériau biologique avec des composants de matrice extracellulaire composite selon la revendication 1, dans lequel le matériel biologique comprend des perforations qui pénètrent dans le matériel biologique.

9. Matériau biologique à composants de matrice extracellulaire composite selon la revendication 8, dans lequel les perforations ont un diamètre de 1 mm à 5 mm, où l'espacement entre les perforations est de 0,5 cm à 5 cm.
